(19) Europäisches Patentamt
European Patent Office
Office européen des brevets

(11) EP 2 257 270 B1

(12) **FASCICULE DE BREVET EUROPEEN**

(45) Date de publication et mention de la délivrance du brevet:
**06.04.2016 Bulletin 2016/14**

(21) Numéro de dépôt: **09710148.9**

(22) Date de dépôt: **06.02.2009**

(51) Int Cl.:
***A61K 8/66** (2006.01)* ***A61K 38/41** (2006.01)*
***A61N 5/06** (2006.01)* ***A61Q 7/00** (2006.01)*
***A61Q 19/08** (2006.01)* ***C07K 14/80** (2006.01)*
***A61K 41/00** (2006.01)*

(86) Numéro de dépôt international:
**PCT/FR2009/050193**

(87) Numéro de publication internationale:
**WO 2009/101344 (20.08.2009 Gazette 2009/34)**

(54) **ASSOCIATION D'UN RAYONNEMENT LUMINEUX ET D'UN SUBSTRAT DE LA CYTOCHROME C OXYDASE POUR AMÉLIORER L'APPARENCE DE LA PEAU ET/OU DU CHEVEU**

KOMBINATION EINES LICHTSTRAHLS MIT EINEM CYTOCHROM-C-OXYDASE-SUBSTRAT ZUR VERBESSERUNG DES AUSSEHENS VON HAUT UND/ODER HAAREN

COMBINATION OF A LIGHT RAY WITH A CYTOCHROME C OXYDASE SUBSTRATE FOR IMPROVING THE APPEARANCE OF THE SKIN AND/OR HAIR

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **06.02.2008 FR 0850758**
**12.02.2008 US 27839 P**

(43) Date de publication de la demande:
**08.12.2010 Bulletin 2010/49**

(73) Titulaire: **L'Oréal**
**75008 Paris (FR)**

(72) Inventeurs:
- **LABOUREAU, Julien**
**92130 Issy les Moulineaux (FR)**
- **NGUYEN, Quang Lan**
**92160 Antony (FR)**
- **PHAM, Dang-Man**
**94370 Sucy-en-brie (FR)**

(74) Mandataire: **Hugodot, Yannick**
**L'Oréal**
**D.I.P.I.**
**25-29 Quai Aulagnier**
**92665 Asnières-sur-Seine Cedex (FR)**

(56) Documents cités:
**EP-A- 1 837 392** **WO-A-03/039478**
**WO-A-2005/011606** **WO-A-2005/058960**
**US-A1- 2005 186 290** **US-A1- 2006 217 690**
**US-A1- 2007 184 003**

- **PERRIN ET AL: "A new cytochrome c-inducing active ingredient that enhances mitochondrial membrane potential and functioning" JOURNAL OF INVESTIGATIVE DERMATOLOGY, [Online] vol. 127, no. 418, 2007, page S70, XP002497798 Extrait de l'Internet: URL:http://www.nature.com/jid/journal/v127/n1s/pdf/5700832a.pdf> [extrait le 2008-09-29]**
- **BAUZA ET AL: "A new active ingredient that improves skin defense and protection by enhancing mitochondrial cytochrome c expression" JOURNAL OF INVESTIGATIVE DERMATOLOGY, [Online] vol. 127, no. 419, 2007, page S70, XP002497799 Extrait de l'Internet: URL:http://www.nature.com/jid/journal/v127/n1s/pdf/5700832a.pdf> [extrait le 2008-09-29]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention relève du domaine du soin de la peau et/ou du cheveu et notamment de l'amélioration de l'apparence de la peau et/ou du cheveu par des rayonnements lumineux.

Par 'peau', on entend la peau du visage et/ou du corps.

La présente invention se rapporte plus spécifiquement à un procédé de traitement cosmétique destiné notamment à améliorer l'apparence de la peau et/ou du cheveu comprenant l'administration simultanée et/ou séquentielle :

- d'au moins un substrat de la cytochrome C oxydase et/ou d'au moins un agent augmentant l'expression dudit substrat; et
- d'au moins un rayonnement lumineux présentant au moins une longueur d'onde prédominante activant la cytochrome C oxydase.

Le procédé de traitement cosmétique selon la présente invention comprend, en particulier, l'administration simultanée et/ou séquentielle, à un sujet, en particulier à un sujet à peau saine :

- d'au moins un substrat de la cytochrome C oxydase et/ou d'au moins un agent augmentant l'expression dudit substrat; et
- d'au moins un rayonnement lumineux présentant au moins une longueur d'onde prédominante activant la cytochrome C oxydase.

Par 'peau saine', on entend une peau ne présentant pas de lésion et/ou d'infection.

En particulier, ledit rayonnement lumineux selon l'invention présente (émet) au moins une longueur d'onde prédominante activant la cytochrome C oxydase allant de 550 à 1000nm (spectre d'émission rouge et infra-rouge), en particulier de 550 à 800nm, de préférence de 620 à 700 nm, et encore plus préférentiellement de 640 à 680nm ; et est utilisé de préférence à une dose allant de 0,01 à 200J/cm$^2$, de préférence de 0,1 à 30J/cm$^2$, plus préférentiellement de 1 à 30J/cm$^2$, voire de 5 à 30J/cm$^2$.

L'invention se rapporte également à une composition comprenant au moins un substrat de la cytochrome C oxydase et/ou un agent augmentant l'expression dudit substrat et au moins un composé présentant et/ou filtrant, notamment sous exposition lumineuse, un rayonnement lumineux présentant au moins une longueur d'onde prédominante activant la cytochrome C oxydase, ainsi qu'à un kit comprenant au moins une composition comprenant au moins un substrat de la cytochrome C oxydase et/ou un agent rayonnement lumineux présentant au moins une longueur d'onde prédominante activant la cytochrome C oxydase, ainsi qu'à un kit comprenant au moins une composition comprenant au moins un substrat de la cytochrome C oxydase et/ou un agent augmentant l'expression dudit substrat et un dispositif et/ou un composé émettant/filtrant un rayonnement lumineux présentant au moins une longueur d'onde prédominante activant la cytochrome C oxydase.

Conserver une apparence jeune et/ou une peau et/ou des cheveux sains conduit à la recherche incessante de nouveaux composés et/ou de nouvelles méthodes non-invasives de traitement permettant de maintenir ou d'améliorer l'apparence de la peau et/ou du cheveu.

On se tourne depuis quelques années vers dés traitements esthétiques mettant en oeuvre des radiations lumineuses de type lumière visible et/ou infrarouge, laser, LED, etc. On parle notamment de 'traitement photocosmétique'.

On connaît notamment l'utilisation par les dermatologues et esthéticiens de lasers et IPL (Intense Pulsed Light) pour traiter les signes du vieillissement cutané, en particulier les rides, mais ces technologies sont susceptibles, dans certaines conditions (forte intensité et/ou longue durée de traitement) de générer des dommages au niveau du derme, pouvant provoquer des érythèmes, des oedèmes ou des douleurs.

On connaît également l'utilisation de la LED (Light-emitting diode) qui active spécifiquement, pour une longueur d'onde donnée, des chromophores ou accepteurs cellulaires dans le tissu cutané, et par là-même initient une cascade de métabolismes biologiques destinés à revitaliser/ améliorer/ régénérer/ stimuler la fonctionnalité et l'apparence de la peau.

La demande WO2004/075985 décrit notamment des méthodes de traitement mettant en oeuvre des radiations lumineuses définies dans le visible et/ou l'infra-rouge, éventuellement en association avec un actif photosensibilisant, pour diminuer les rides, rajeunir la peau, favoriser la cicatrisation et la réparation des tissus, traiter les désordres hypopigmentaires. Il est décrit l'application répétée et contrôlée en plusieurs étapes de radiations lumineuses, sur dès périodes de plusieurs heures.

Il reste le besoin de développer des systèmes qui soient faciles à mettre en oeuvre et ne présentant pas les dommages cutanés susceptibles d'être générés par une application particulier prévenir et/ou améliorer l'état de surface des gerçures et/ou des gelures, liées par exemple à une déficience d'oxygénation cellulaire (situations cellulaires ischémiques causées notamment par le froid et l'altitude).

Selon un mode particulier, le procédé selon l'invention vise à améliorer l'hydratation de la peau et/ou du cheveu.

Selon un autre mode, le procédé selon l'invention vise à améliorer la fonction barrière cutanée.

Selon encore un autre mode, le procédé selon l'invention vise à favoriser la régénération des cellules dermiques et/ou épidermiques, en particulier améliorer l'état de surface des gerçures et/ou gelures.

Le procédé selon l'invention vise encore à prévenir et/ou lutter contre les signes du vieillissement de la peau et/ou du cheveu.

En particulier, le procédé selon l'invention vise à atténuer les irrégularités visibles ou tactiles de la surface de la peau, en particulier atténuer les rides et ridules, atténuer les tâches cutanées, diminuer les altérations du microrelief et/ou lisser la peau, favoriser la régénération du tissu cutané, éclaircir le teint et/ou améliorer l'aspect terne du teint, maintenir et/ou améliorer les propriétés biomécaniques de la peau (élasticité, fermeté, tonicité), maintenir et/ou améliorer l'hydratation de la peau, et/ou maintenir et/ou améliorer le grain de la peau.

Il vise également à traiter le ralentissement de la pousse des cheveux, leur grisonnement, la diminution de leur diamètre, et de leur vigueur.

## SUBSTRATS DE LA CYTOCHROME C OXYDASE ET AGENTS AUGMENTANT

## L'EXPRESSION DUDIT SUBSTRAT

Comme substrat de la cytochrome C oxydase, on entend notamment les dérivés de composés ayant un site catalytique protoporphyrinique IX contenant du fer.

**<u>Schéma de la réaction conduisant à la production d'ATP et de molécules d'eau à partir d'oxygène</u>**

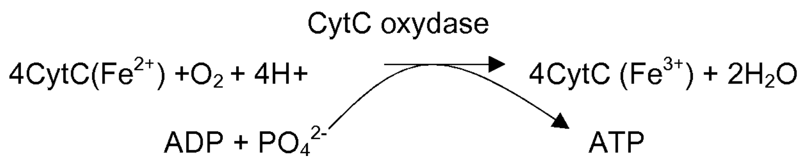


Le Cytochrome C, un de ses substrats, est une petite protéine hème ayant un site catalytique protoporphyrinique IX contenant du fer associée avec la membrane interne de la mitochondrie. C'est une protéine soluble, à l'inverse des autres cytochromes. Elle est un composant essentiel de la chaîne respiratoire. Le cytochrome C est une protéine hautement conservée à travers le spectre des espèces, trouvée dans les plantes, les animaux et de nombreux organismes unicellulaires. On lui connaît des analogues, connus sous les dénominations cytochromes C1, C2 et C3.

On sait par ailleurs que le nombre de mutations au niveau de l'ADN mitochondrial des cellules cutanées, en particulier des fibroblastes, augmente avec l'âge, notamment au niveau de séquences codantes pour la cytochrome oxydase (G. S. Gerhard et al., Mechanisms of Ageing and development, 123, 2002 : 155-166), ayant pour effet d'altérer la production d'énergie et de diminuer le métabolisme global des cellules de la peau.

Les rayonnements UV sont également responsables d'une augmentation des mutations au niveau de l'ADN du génome mitochondrial, notamment dans des séquences d'ADN codant pour des protéines impliquées dans la chaîne respiratoire de la mitochondrie. L'altération de ces protéines conduit à une diminution de la consommation d'oxygène, une variation du potentiel membranaire, une diminution de la production d'ATP et une augmentation de la synthèse des MMPs. Ces mécanismes se traduisent notamment par une perte d'hydratation au niveau des cellules de la peau, une dégradation du collagène et une diminution du métabolisme énergétique des cellules.

L'association selon l'invention permet donc de maintenir le stock énergétique (ATP) des cellules et une production de molécules d'eau pour que l'ensemble des fonctions cellulaires soit assuré.

L'invention porte donc sur l'association d'au moins un substrat de la cytochrome C oxydase et/ou d'au moins un agent augmentant l'expression dudit substrat avec au moins un rayonnement lumineux présentant au moins une longueur d'onde prédominante activant la cytochrome C oxydase, en particulier un rayonnement lumineux présentant au moins une longueur d'onde prédominante allant de 550 à 1000nm (spectre d'émission de la lumière rouge et/ou infra-rouge), dans un procédé de traitement cosmétique, une composition, ou un kit destinés notamment à améliorer l'apparence de la peau et/ou du cheveu.

Le substrat de la cytochrome C oxydase et/ou l'agent augmentant l'expression dudit substrat peuvent être formulés dans une composition pour administration par voie topique.

Le rayonnement lumineux présentant au moins une longueur d'onde prédominante activant la cytochrome C oxydase, peut être sous la forme d'un dispositif, ou selon une alternative, sous la forme d'un composé capable d'émettre

et/ou de laisser spécifiquement passer, notamment sous exposition lumineuse, un rayonnement lumineux présentant au moins une longueur d'onde prédominante activant la cytochrome C oxydase. Dans ce dernier cas, ledit composé émettant et/ou filtrant ledit rayonnement lumineux activant la cytochrome C oxydase peut être formulé dans la même composition que le substrat de la cytochrome C oxydase et/ou l'agent augmentant l'expression dudit substrat, ou dans une composition distincte.

L'invention porte donc notamment sur un procédé cosmétique destiné notamment à améliorer l'apparence de la peau et/ou du cheveu comprenant l'administration simultanée et/ou séquentielle :

a) d'au moins un substrat de la cytochrome C oxydase et/ou d'au moins un agent augmentant l'expression dudit substrat;
b) d'au moins un rayonnement lumineux présentant au moins une longueur d'onde prédominante activant la cytochrome C oxydase.

De façon avantageuse, tous les procédés cosmétiques selon la présente invention sont mis en oeuvre sur des sujets à peau saine.

Par 'rayonnement lumineux présentant au moins une longueur d'onde prédominante', on entend selon l'invention un rayonnement lumineux distinct de la lumière blanche comprenant toutes les longueurs d'onde du spectre.
On sait en effet que chaque longueur d'onde a une cible spécifique dans les cellules de la peau et/ou du cheveu, appelée chromophore ou photoaccepteur.
La Demanderesse a en effet montré que certaines longueurs d'onde étaient capables d'activer la cytochrome C oxydase, alors que d'autres étaient sans effet, voire étaient susceptibles de l'inhiber.

De préférence, le rayonnement lumineux présente au moins une longueur d'onde prédominante allant de 550 à 1000nm (spectre d'émission de la lumière rouge 550-800nm et infra-rouge 800-1000nm).
En particulier, le rayonnement lumineux présente au moins une longueur d'onde prédominante allant de 550 à 800nm (spectre d'émission de la lumière rouge), en particulier de 620 à 700 nm, et encore plus préférentiellement de 640 à 680nm. En particulier, on utilisera un rayonnement lumineux dont le spectre d'émission a un pic optimal autour de 660nm, de préférence autour de 660 nm +/- 100 nm, en particulier autour de 660 nm +/- 40 nm, et de manière tout à fait préférée autour de 660 nm +/- 15 nm.

Selon un mode préféré, le rayonnement lumineux présentant au moins une longueur d'onde prédominante activant la cytochrome C oxydase est appliqué sur la peau et/ou le cheveu, en particulier au niveau des zones de peau et/ou de cheveu traitées par une composition comprenant au moins un substrat de la cytochrome C oxydase et/ou un agent augmentant l'expression dudit substrat, à une dose allant de 0,01 à 200J/cm2, de préférence de 0,1 à 30J/cm2.

Par 'séquentielle', on entend une administration successive (immédiate) ou retardée. Dans le cas d'une administration séquentielle et de façon avantageuse, on administre le substrat de la cytochrome C oxydase et/ou l'agent augmentant l'expression dudit substrat avant l'administration du rayonnement lumineux présentant au moins une longueur d'onde prédominante activant la cytochrome C oxydase, mais ledit substrat et/ou ledit agent augmentant l'expression dudit substrat peut également et de façon complémentaire être administré après une session substrat + rayonnement lumineux présentant au moins une longueur d'onde prédominante activant la cytochrome C oxydase.

Selon un mode particulier, le rayonnement lumineux présentant au moins une longueur d'onde prédominante activant la cytochrome C oxydase est un composé émettant et/ou filtrant, notamment sous exposition lumineuse ou UV, ledit rayonnement lumineux activant la cytochrome C oxydase ; ledit composé peut être formulé dans la composition comprenant ledit substrat de la cytochrome C oxydase et/ou ledit agent augmentant l'expression dudit substrat ou bien dans une autre composition topique destinée à être appliquée de façon décalée (avant ou après).

Selon un autre mode, on utilise un patch, notamment iontophorétique, imbibé d'au moins un substrat de la cytochrome C oyxdase et/ou d'un agent augmentant l'expression dudit substrat et muni de diodes émettant ledit rayonnement lumineux activant la cytochrome C oxydase.

La mise en oeuvre du procédé selon l'invention pourra en outre comprendre une étape préalable ou concomitante aux étapes du procédé selon l'invention, visant à améliorer la pénétration dudit substrat de la cytochrome C oxydase et/ou d'un agent augmentant l'expression dudit substrat, par exemple en refroidissant la peau, par iontophorèse, ou par un système occlusif.

On peut également favoriser la pénétration du substrat de la cytochrome C oxydase et/ou d'un agent augmentant l'expression dudit substrat, en pratiquant un peeling chimique ou mécanique préalable sur la zone à traiter.

Une telle association a pour effet d'assurer le bon fonctionnement de la chaîne respiratoire mitochondriale, et permet ainsi d'augmenter la production d'énergie et de lutter notamment contre les signes du vieillissement et de déshydratation cellulaire au niveau de la peau et/ou du cheveu.

L'association selon l'invention permet donc notamment de maintenir et/ou améliorer le métabolisme énergétique des cellules de la peau et/ou du cheveu ; maintenir et/ou améliorer l'hydratation de la peau et/ou du cheveu ; maintenir et/ou améliorer les propriétés biomécaniques de la peau (élasticité, fermeté, tonicité) ; prévenir et/ou traiter les signes

du vieillissement de la peau et/ou du cheveu ; améliorer la fonction barrière cutanée ; favoriser la régénération des cellules dermiques et/ou épidermiques, en particulier prévenir et/ou améliorer l'état de surface des gerçures et/ou des gelures, liées par exemple à une déficience d'oxygénation cellulaire (situations cellulaires ischémiques causées notamment par le froid et l'altitude).

Selon un mode particulier, le procédé selon l'invention vise à améliorer l'hydratation de la peau et/ou du cheveu.

Selon un autre mode, le procédé selon l'invention vise à améliorer la fonction barrière cutanée.

Selon encore un autre mode, le procédé selon l'invention vise à favoriser la régénération des cellules dermiques et/ou épidermiques, en particulier améliorer l'état de surface des gerçures et/ou gelures.

Le procédé selon l'invention vise encore à prévenir et/ou lutter contre les signes du vieillissement de la peau et/ou du cheveu.

En particulier, le procédé selon l'invention vise à atténuer les irrégularités visibles ou tactiles de la surface de la peau, en particulier atténuer les rides et ridules, atténuer les tâches cutanées, diminuer les altérations du microrelief et/ou lisser la peau, favoriser la régénération du tissu cutané, éclaircir le teint et/ou améliorer l'aspect terne du teint, maintenir et/ou améliorer les propriétés biomécaniques de la peau (élasticité, fermeté, tonicité), maintenir et/ou améliorer l'hydratation de la peau, et/ou maintenir et/ou améliorer le grain de la peau.

Il vise également à prévenir et/ou traiter la chute des cheveux, le ralentissement de leur pousse, leur grisonnement, la diminution de leur diamètre, de leur vigueur.

**SUBSTRATS DE LA CYTOCHROME C OXYDASE ET AGENTS AUGMENTANT**

**L'EXPRESSION DUDIT SUBSTRAT**

Comme substrat de la cytochrome C oxydase, on entend notamment les dérivés de composés ayant un site catalytique protoporphyrinique IX contenant du fer.

A titre d'exemple selon l'invention, on peut citer notamment la cytochrome C, ses analogues, tels que la cytochrome C1, la cytochrome C2, la cytochrome C3, et leurs mélanges.

Selon un procédé de l'invention, le substrat de la cytochrome C oxydase est choisi parmi la cytochrome C, l'un de ses analogues cytochromes C1, C2 ou C3, et leurs mélanges.

La cytochrome C est notamment commercialisée par SIGMA, par exemple dans un kit l'associant à une cytochrome C oxydase vendu sous la dénomination Cytocox 1.

Comme alternative à l'utilisation d'un substrat de la cytochrome C oxydase, ou en complément, on pourra utiliser un agent augmentant l'expression dudit substrat de la cytochrome C oxydase.

Cette alternative peut être avantageuse pour répondre à des problématiques éventuelles de formulation du substrat de la cytochrome C oxydase.

On entend par « agent augmentant l'expression dudit substrat de la cytochrome C oxydase », tout agent (ou substance) capable d'augmenter l'expression dudit substrat de la cytochrome C oxydase, notamment, par activation de la synthèse protéique du substrat de la Cytochrome C oxydase (en particulier *via* une régulation du gène codant pour ledit substrat).

On entend par « agent augmentant l'expression de la cytochrome C » tout agent (ou substance) capable d'augmenter l'expression de la cytochrome C notamment, par activation de la synthèse protéique de la Cytochrome C (en particulier *via* une régulation du gène codant pour la protéine Cytochrome C.

Comme agent augmentant l'expression dudit substrat de la cytochrome C oxydase, on peut citer un agent augmentant l'expression de la cytochrome C.

D'autres agents augmentant l'expression de la cytochrome C, utilisables selon l'invention, peuvent être sélectionnés par l'homme du métier, par exemple dans un test in *vitro* sur fibroblastes humains consistant à :

- incuber des fibroblastes dans un milieu de culture adapté à leur croissance et leurs activités de synthèse ;
- les mettre en présence ou non (témoin) d'une quantité suffisante du composé à tester, par exemple 0.1, 1, 3% ;
- mesurer par marquage immunofluorescent sur culture cellulaire et/ou Western Blot en utilisant un anticorps spécifique pour le cytochrome C, l'expression de la cytochrome C induite par la présence du composé à tester ;
- sélectionner les composés pour lesquels on obtient une augmentation de l'expression de la cytochrome C par rapport au témoin, de préférence une augmentation de plus de 10%, voire de plus de 20% par rapport au témoin, en particulier lorsque le composé est ajouté à la concentration de 1% dans le milieu de culture.

Selon un premier mode de réalisation, le substrat de la cytochrome C oxydase et/ou l'agent augmentant l'expression dudit substrat est formulé dans une composition destinée à une administration par voie topique sur la peau.

Il pourra être encore avantageux de combiner des administrations par voie topique pour optimiser les effets recherchés.

Ledit substrat de la cytochrome C oxydase peut être présent dans une composition selon l'invention en une teneur en matière active allant de 0,000001 à 20% en poids par rapport au poids total de ladite composition.

Ledit substrat de la cytochrome C oxydase sera généralement présent dans une composition de l'invention en une teneur allant de 0,001 à 20% en poids par rapport au poids total de ladite composition. De préférence, la teneur ira de 0,01 à 10% en poids, et préférentiellement de 0,01 à 1% en poids par rapport au poids total de ladite composition. De préférence, ledit substrat de la cytochrome C oxydase sera présent dans une composition selon l'invention en une teneur en matière active allant de 0,00001 à 2%, et de manière tout à fait préférée, ledit substrat de la cytochrome C oxydase sera présent dans une composition selon l'invention en une teneur en matière active allant de 0,00002% à 0,1%.

L'agent augmentant l'expression dudit substrat de la cytochrome C oxydase peut être présent dans une composition selon l'invention en teneur en matière active allant de 0,000001 à 30% en poids par rapport au poids total de ladite composition.

L'agent augmentant l'expression dudit substrat de la cytochrome C oxydase sera généralement présent dans une composition de l'invention en une teneur allant de 0,001 à 30% en poids par rapport au poids total de ladite composition. De préférence, la teneur ira de 0,01 à 20% en poids, et préférentiellement de 0,01 à 5% en poids par rapport au poids total de ladite composition.

De préférence, ledit agent augmentant l'expression dudit substrat de la cytochrome C oxydase sera présent dans une composition selon l'invention en une teneur en matière active allant de 0,00001 à 2%, et de manière tout à fait préférée, ledit substrat de la cytochrome C oxydase sera présent dans une composition selon l'invention en une teneur en matière active allant de 0,00002% à 2%.

Les teneurs en poids les plus élevées sont généralement réservées à des applications sous forme solide (ex : poudre) ou de patch.

## RAYONNEMENT LUMINEUX ACTIVANT LA CYTOCHROME C OXYDASE

Par 'rayonnement lumineux activant la cytochrome C oxydase', on entend un rayonnement lumineux utilisé dans des conditions (longueur d'onde, intensité, durée d'exposition) telles qu'elles puissent stimuler l'activité enzymatique de la cytochrome C oxydase en présence de son substrat et/ou d'un agent augmentant l'expression dudit substrat, et notamment stimuler la réaction suivante conduisant à une production d'énergie (ATP) et de molécules d'eau.

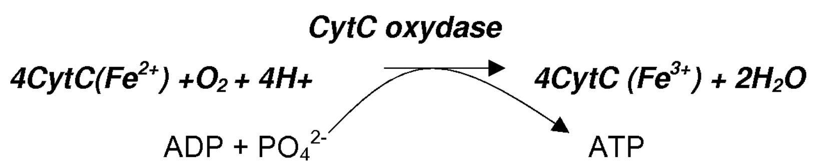


Un rayonnement lumineux activant la cytochrome C oxydase, utilisable selon l'invention, peut être sélectionné selon un test *in vitro* tel que décrit à l'exemple 1 ci-après, consistant à:

- exposer ou non (témoin) la cytochrome C oxydase et son substrat (cytochrome C), à des rayonnements lumineux de longueurs d'onde données, par exemple à une dose de 2,7Joule,
- évaluer l'effet dudit rayonnement lumineux sur l'activité de l'enzyme, par rapport à un témoin, et
- sélectionner le rayonnement lumineux capable d'augmenter l'activité de ladite enzyme, en particulier d'augmenter d'au moins 10% l'activité de ladite enzyme par rapport au témoin maintenue à l'abri de la lumière.

La Demanderesse a en effet pu montrer que si un rayonnement lumineux présentant au moins une longueur d'onde prédominante correspondant au spectre d'émission de la lumière rouge (dose 2,7 Joule) était capable d'activer la cytochrome C oxydase (+33%), d'autres longueurs d'onde (ex : lumière verte autour de 535nm et la lumière bleue autour de 447nm) n'avaient pas d'effet.

Lorsque le rayonnement lumineux est absorbé par un tissu, il délivre une énergie au tissu, qui lui-même réagit au rayonnement lumineux en fonction de la longueur d'onde, de l'intensité et du temps d'exposition audit rayonnement lumineux.

L'intensité du rayonnement lumineux utilisée selon l'invention ne dépasse pas de préférence 150mW/cm$^2$, et de préférence est supérieure à 0,01mW/cm$^2$. Cette plage d'intensité assure une efficacité de traitement sur une durée raisonnable, sans causer de dommages au tissu cutané traité.

La durée d'exposition au rayonnement lumineux activant la cytochrome C oxydase sera définie, en fonction de l'intensité dudit rayonnement, de sorte à ce que l'énergie totale délivrée (dose) sur la peau et/ou le cheveu soit de 0,01 à 200J/cm$^2$, de préférence de 0,1 à 30J/cm$^2$, plus préférentiellement de 1 à 30J/cm$^2$, voire de 5 à 30J/cm$^2$.

Energie (en Joule) = Intensité (en Watt) x Temps (en seconde)

A titre non limitatif, le temps d'exposition à un dispositif émettant un rayonnement lumineux activant la cytochrome C oxydase pourra aller de 20mn à 120mn, de préférence de 30mn à 120mn et encore plus préférentiellement de 60mn à 90mn.

En fonction de l'état de surface de la peau et/ou du cheveu à traiter et de l'effet recherché, il pourra être avantageux de procéder à plusieurs applications par jour dudit rayonnement lumineux activant la cytochrome C oxydase en association avec le substrat de la cytochrome C oxydase, ou d'une application par jour, voire d'une application par semaine, pendant une durée de un à plusieurs mois.

Selon un mode préféré, le rayonnement lumineux activant la cytochrome C oxydase sera notamment un rayonnement lumineux présentant au moins une longueur d'onde prédominante allant de 550 à 1000nm, de préférence de 550 à 800nm, en particulier de 620 à 700 nm, et encore plus préférentiellement de 640 à 680nm ; utilisé de préférence à une dose allant de 0,01 à 200J/cm$^2$, de préférence de 0,1 à 30J/cm$^2$, plus préférentiellement de 1 à 30J/cm$^2$, voire de 5 à 30J/cm$^2$.

Selon un mode préféré, le rayonnement lumineux présentant au moins une longueur d'onde prédominante activant la cytochrome C oxydase est ainsi appliqué sur la peau et/ou le cheveu, en particulier au niveau des zones de peau et/ou de cheveu traitées par la composition comprenant au moins un substrat de la cytochrome C oxydase et/ou un agent augmentant l'expression de la cytochrome C, à une dose allant de 0,01 à 200J/cm$^2$, de préférence de 0,1 à 30J/cm$^2$.

Comme 'rayonnement lumineux présentant au moins une longueur d'onde prédominante activant la la cytochrome C oxydase utilisable selon l'invention, on peut notamment utiliser :

- un rayonnement lumineux émis par un dispositif (source 'physique'),
- un rayonnement lumineux émis par un composé (source 'chimique/biologique'),

et leurs mélanges.

<u>**Dispositifs émettant le rayonnement lumineux activant la cytochrome C oxydase**</u>

Selon un premier mode de réalisation de l'invention, le rayonnement lumineux présentant au moins une longueur d'onde prédominante activant la cytochrome C oxydase est émis par un dispositif.

Ledit dispositif pourra notamment être choisi parmi un dispositif émettant de la lumière blanche associée à un filtre spécifique laissant passer ledit rayonnement activant la cytochrome C oxydase ; les lasers ; l'IPL ; les LED ; et leurs combinaisons.

En particulier, ledit dispositif émet un rayonnement lumineux présentant au moins une longueur d'onde prédominante allant de 550 à 1000nm, en particulier de 550 à 800 nm, choisi notamment parmi : les lasers, l'IPL, les LED ; un dispositif émettant de la lumière blanche associée à un filtre spécifique laissant passer au moins une longueur d'onde prédominante allant de 550 à 1000nm (spectre d'émission du rouge et/ou de l'infra-rouge), et leurs combinaisons.

Comme dispositifs émettant un rayonnement lumineux activant la cytochrome C oxydase utilisables selon l'invention, on peut notamment citer :

- <u>la lumière blanche</u>, naturelle ou artificielle, associée à un filtre spécifique ou un dispositif écran laissant passer au moins une longueur d'onde prédominante activant la cytochrome C oxydase (ex : longueur d'onde du spectre d'émission du rouge et/ou de l'infra-rouge): on peut citer notamment les lampes à ARC (ex : lampe Xenon), les lampes à incandescence comme exemples de dispositifs émettant de la lumière blanche artificielle. Par lumière blanche naturelle, on entend la lumière du jour, que l'on associera à un dispositif écran laissant passer spécifiquement de façon prédominante une couleur (ex : rouge) activant la cytochrome C oxydase.

- <u>les lasers</u> (pour Light Amplification by the Stimulated Emission of Radiation), il s'agit d'une source lumineuse de très haute intensité et monochromatique.

A la différence de la lumière blanche (photons de diverses longueurs d'onde émis de façon anarchique à différents instants et dans des directions différentes), la radiation lumineuse émise par un laser est une lumière constituée de photons émis en même temps et dans la même direction.

Trois éléments caractérisent les lasers : la longueur d'onde ($\lambda$) ; le mode d'émission : continu (puissance délivrée constante), pulsé (l'énergie est délivrée par pulses dont la fréquence et la puissance sont modulables), et ultra-pulsé (les pulses ont une durée et une puissance fixe, mais la puissance est considérable et la durée extrêmement brève) ; et la puissance : de quelques mW à des dizaines de milliers de watts.

- l'IPL : Intense pulse light.

La différence fondamentale entre laser et IPL réside dans le fait que IPL peut délivrer des centaines voire des milliers de couleurs à la fois alors que le laser ne délivre qu'une seule longueur d'onde. Ces machines permettent de choisir la longueur d'onde adaptée au problème à traiter, rien qu'en changeant le filtre. On les appelle aussi « non coherent light source ».
Il s'agit d'émission de pulses de lumière de haute intensité globale.
Cette technologie peut délivrer un large spectre d'émission de longueurs d'onde qui sont absorbées par de multiples chromophores. On peut traiter de grandes surfaces à la fois.

- les LED : Light emitting diode (Photomodulation (LED, Light Emitting Diode voir "The newest Médical breakthrough for skin renewal and Shrinking pores (2004)").
La LED : la LED émet généralement de la lumière à basse intensité de quelques milliwatt ; on les classe dans la catégorie des lasers à faible puissance (1 à quelques dizaines de mW).

Selon un mode préféré, on utilisera des dispositifs de type LED.
Comme exemples de dispositifs de type LED émettant un rayonnement lumineux activant la cytochrome C oxydase disponibles commercialement, on peut citer notamment :

a) des dispositifs susceptibles d'être utilisés notamment dans des instituts de soin de la peau et/ou du cheveu, tels que :

- Omnilux™ system (633nm) de la société Photo Therapeutics Ltd.;
- Lumiphase™ system (660nm) de la société OPUSMED Inc ;
- Lightwave system (630 nm et IR 880nm) de la société Lightwave Technologies ;
- Delphia™ et HydroFacial™ (600-700nm et IR 700-1000nm) systems de la société Edge Systems Corp ;
- RevitaLight system (625nm ; IR 940nm) de la société Skincare Systems Inc.

b) des dispositifs susceptibles d'être utilisés chez soi, tels que ceux commercialisés notamment sur internet (sites spécialisés ou sites de vente, tels que e-Bay) ; on peut citer par exemple le Mini photon de Photo Rejuvenation.

Le rayonnement lumineux selon l'invention est de préférence substantiellement monochromatique (longueur d'onde prédominante) avec une longueur d'onde allant de 550 à 1000nm, de préférence de 550 à 800nm, en particulier allant de 620 à 700nm, encore plus préférentiellement de 640 à 680nm, et mieux encore autour de 660nm.
Selon un mode particulier, il pourra être avantageux d'avoir un dispositif émettant séquentiellement un rayonnement lumineux vert et un rayonnement lumineux rouge ou infra-rouge, pour complémenter les effets anti-âge de l'une et l'autre de ces lumières. Dans ce cas, les sessions de traitement au rayonnement lumineux rouge seront de préférence décalées dans le temps par rapport aux sessions de traitement au rayonnement vert, pour éviter toute interférence entre les effets des différentes longueurs d'onde.
L'application séquentielle de deux dispositifs émettant à des longueurs d'onde différentes permet ainsi de tirer partie des bénéfices de chaque lumière sans interférence de l'une avec l'autre.

**Composés émettant un rayonnement lumineux activant la cytochrome C oxydase**

Selon un autre mode de réalisation de l'invention, ou en complément d'un dispositif tel que décrit précédemment, le rayonnement lumineux présentant au moins une longueur d'onde prédominante activant la cytochrome C oxydase est émis par un composé choisi parmi les métabolites ou actifs émettant, notamment sous exposition lumineuse (visible, UV), une telle longueur d'onde prédominante activant la cytochrome C oxydase, un composé filtrant la lumière pour laisser spécifiquement passer la ladite longueur d'onde prédominante activant la cytochrome C oxydase, et leurs mélanges.
Selon un mode préféré, ledit composé émet un rayonnement lumineux présentant au moins une longueur d'onde prédominante activant la cytochrome C oxydase allant de 550 à 1000nm, de préférence de 550 à 800nm, en particulier de 620 à 700nm, et plus préférentiellement encore de 640 à 680nm..
Selon un mode particulier, ledit composé émet ledit rayonnement lumineux lorsqu'il est sous exposition lumineuse (visible, UV). Cette exposition lumineuse peut être une exposition à la lumière du jour (lumière 'naturelle') ou une exposition à une lumière artificielle mettant en oeuvre un dispositif. En particulier, on utilisera un dispositif émettant une lumière blanche ou colorée ou UV destinée à exciter ledit composé pour lui permettre d'émettre ladite longueur d'onde prédominante (ex : rouge).

L'intensité de cette exposition lumineuse sera telle qu'elle permette audit composé d'émettre ledit rayonnement activant la cytochrome C oxydase, en particulier à une dose allant de 0,01 à 200J/cm$^2$, de préférence de 0,1 à 30J/cm$^2$, plus préférentiellement de 1 à 30J/cm$^2$, voire de 5 à 30J/cm$^2$.

Selon un autre mode, ledit composé émet un rayonnement lumineux sans exposition à la lumière : c'est le cas des composés à réaction exothermique (thermoluminescence), ou des composés à réaction de chimiluminescence ou de bioluminescence.

Selon une variante, ledit composé émet un rayonnement lumineux, notamment de type infra-rouge, *via* une réaction exothermique ou de thermoluminescence (dégagement de chaleur) ; par exemple en solubilisant du CaCl2 (chlorure de calcium), du ZnCl2 (chlorure de zinc), ou du AlCl3 (Chlorure d'aluminium). Cette réaction peut avoir lieu au moment de l'application sur la peau d'une composition anhydre contenant ledit composé, la peau contenant intrinsèquement une quantité d'eau suffisante pour déclencher la réaction exothermique.

Selon une autre variante, ledit composé émet un rayonnement lumineux par réaction de chimiluminescence ou de bioluminescence.

On peut citer notamment la réaction de bioluminescence de la luciférine en présence de l'enzyme luciférase, d'ATP, d'un minéral (ex : le magnésium) et de dioxygène, qui existe de façon naturelle chez les lucioles :

$$\text{luciferin} + \underline{\text{ATP}} + \underline{O_2} \rightarrow \text{oxyluciferin} + \underline{\text{AMP}} + \underline{PP_i} + \text{lumière}$$

Le composé peut encore être capable de filtrer le rayonnement lumineux activant la cytochrome C oxydase selon l'invention, lorsqu'il est soumis à une exposition lumineuse, naturelle ou artificielle.

Ces phénomènes chromiques et les composés correspondant sont notamment décrits dans l'ouvrage de référence 'Chromic Phenomena, Technological Applications of Colour Chemistry, Peter Bamfield, The Royal Society of Chemistry, 2001'.

*Métabolites et actifs émettant et/ou filtrant la lumière rouge*

Ces substances sont plus particulièrement des métabolites ou actifs émettant, notamment sous exposition lumineuse (visible, UV), de la lumière entre 550 à 800nm, de préférence de 620 à 700nm et encore plus préférentiellement de 640 à 680nm.

On peut citer notamment :

- des anthocyanes de couleur rouge ; des pigments ou colorants rouges ; des complexes de fer III ;
- des substances phosphorescentes ;
- des substances fluorescentes ;

et leurs mélanges.

A titre d'exemple, on peut citer notamment les anthocyanes de couleur rouge tels que la carthamine, la cyanidine, la delphinidine la pélargonine et la bétanine; les polyphénols rouges; le lycopène ; la braziléine, l'extrait de Rocou, la Garance, les pigments rouges, les complexes de fer III, et leurs mélanges.

De préférence, on utilisera le lycopène, la delphinidine et leurs mélanges.

Comme pigments rouges, on peut citer notamment l'acide carminique, l'acide laccaique, les phycoérythrines (des algues), et leurs mélanges.

Commes complexes de fer III, on peut citer notamment les complexes avec l'acide salicylique et dérivés (salicylates), avec des antibactériens tels que l'octopirox, avec le thiocyanate, et leurs mélanges.

Comme substances phosphorescentes, excitables sous lumière visible ou UV, on peut citer notamment des pigments phosphorescents.

Par "pigments phosphorescents rouges", on entend les pigments phosphorescents classiques listés ci-après mais aussi toute substance cosmétiquement acceptable qui émet un rayonnement lumineux rouge phosphorescent d'une longueur d'onde comprise entre environ 550 à 800nm, de préférence de 620 à 700nm et encore plus préférentiellement de 640 à 680nm.

La présence de pigments phosphorescents fournit une source continue de lumière rouge à la composition, la phosphorescence est activée par l'exposition aux UV classiquement présent dans la lumière du jour et son effet dure plusieurs heures.

Comme substances fluorescentes, excitables sous lumière visible, UV ou proche IR, on peut citer notamment des pigments fluorescents tels que le Nil Red (excitation 552nm/ émission 636nm) ; les dérivés de porphyrines glycoconjugués ou non (excitation 420-440/ émission 650-680nm), et leurs mélanges.

En particulier, les métabolites ou actifs émettant de la lumière entre 550 et 800 nm notamment sous exposition lumineuse, pourront être choisis parmi les anthocyanes de couleur rouge, tels que la carthamine, la cyanidine, la delphinidine, la pelargonine, la bétanine ; les polyphénols de couleur rouge ; le lycopène ; la braziléine, l'extrait de Rocou, la Garance, les pigments rouges, les complexes de fer III avec l'acide salicylique, l'octopirox ou le thiocyanate, les substances phosphorescentes rouges, les substances fluorescentes rouges, et leurs mélanges.
De préférence, on utilisera les anthocyanes de couleur rouge et/ou le lycopène.

*Autres composés filtrant la lumière rouge*

On peut encore utiliser, de façon alternative et/ou en association avec les composés émettant un rayonnement lumineux activant la cytochrome C oxydase décrits précédemment, des composés capables de filtrer la lumière pour laisser passer spécifiquement le rayonnement lumineux activant la cytochrome C oxydase.

A titre d'exemple, on peut citer les pigments holographiques comportant une matrice polymérique dans laquelle un holograme volumique est enregistré, qui agissent comme des prismes et ont pour propriété, en choisissant l'indice de réfraction adapté, d'émettre de la lumière rouge lorsqu'ils sont éclairés par de la lumière blanche. Ces systèmes sont décrits par la Demanderesse dans la demande FR0758017.
L'hologramme volumique convertit la lumière incidente en un ou plusieurs faisceaux ayant des dépendances spatiales et spectrales fonction des paramètres de l'hologramme.
Un exemple d'hologramme volumique est l'hologramme de type « Denisyuk » qui est un hologramme de réflexion pouvant être obtenu dans un film holographique à l'aide d'un faisceau laser unique, servant à la fois de faisceau objet et de faisceau de référence, comme divulgué dans la demande EP 1 754 968 A2 et sur le site http://www.smartholograms.com/site/sections/technology/creating-sensors.htm, et dans la demande EP 1 369 681 A1 (voir figures 1a et 1 b notamment), ces publications étant incorporées par référence.

Ces composés filtrant la lumière rouge requièrent une exposition à la lumière, naturelle ou artificielle, pour laisser passer spécifiquement le rayonnement lumineux activant la cytochrome C oxydase.

Ces composés émettant et/ou filtrant le rayonnement lumineux activant la cytochrome C oxydase peuvent être utilisés à des concentrations suffisantes pour émettre et/ou assurer le passage d'une quantité de rayonnement lumineux activant la cytochrome C oxydase.

Ledit composé émettant et/ou filtrant, notamment sous exposition lumineuse, un rayonnement lumineux présentant au moins une longueur d'onde prédominante activant la cytochrome C oxydase, est présent dans l'une au moins des compositions mises en oeuvre dans le procédé de l'invention, en une teneur allant de 0,01 à 20% en poids par rapport au poids total de ladite composition, de préférence de 0,1% à 10% en poids par rapport au poids total de ladite composition.

Selon un premier mode, ledit composé émettant et/ou filtrant, notamment sous exposition lumineuse, un rayonnement lumineux présentant au moins une longueur d'onde prédominante activant la cytochrome C oxydase, est présent dans la composition comprenant ledit substrat de cytochrome C oxydase et/ou l'agent augmentant l'expression dudit substrat.

Selon un mode alternatif, ledit composé émettant et/ou filtrant, notamment sous exposition lumineuse, un rayonnement lumineux présentant au moins une longueur d'onde prédominante activant la cytochrome C oxydase, est présent dans une composition distincte de la composition comprenant ledit substrat de cytochrome C oxydase et/ou l'agent augmentant l'expression dudit substrat.

Ces composés émettant et/ou filtrant le rayonnement lumineux activant la cytochrome C oxydase sont utilisés en quantité suffisante pour assurer un flux d'émission de lumière de 0,1 à quelques dizaines de mW.
L'homme du métier adaptera la durée d'exposition à ce rayonnement lumineux émis par ledit composé selon les caractéristiques dudit composé et l'effet recherché.

A titre purement indicatif, les zones de peau et/ou du cheveu à traiter pourront recevoir une énergie totale (dose) délivrée sur la peau et/ou le cheveu allant de 0,01 à 200J/cm$^2$, de préférence de 0,1 à 30J/cm$^2$, plus préférentiellement de 1 à 30J/cm$^2$, voire de 5 à 30J/cm$^2$.

Des exemples de substrats de la cytochrome C oxydase sont décrits précédemment. En particulier, le substrat de la cytochrome C oxydase est choisi parmi la cytochrome C, l'un de ses analogues cytochromes C1, C2 ou C3, et leurs mélanges.

Selon un mode particulier, le substrat de la cytochrome C oxydase et/ou l'agent augmentant l'expression dudit substrat est formulé dans une composition destinée à une administration par voie topique sur la peau.

Selon un autre mode, le substrat de la cytochrome C oxydase et/ou l'agent augmentant l'expression dudit substrat est formulé dans une composition destinée à une administration par voie orale.

De préférence, on utilisera une administration par voie topique.

En particulier, ledit substrat de la cytochrome C oxydase est présent dans la composition en une teneur allant de 0,001 à 20% en poids par rapport au poids total de ladite composition.

Et l'agent augmentant l'expression dudit substrat de la cytochrome oxydase est présent dans la composition de préférence en une teneur allant de 0,001 à 30% en poids par rapport au poids total de ladite composition.

L'invention a encore pour objet une composition comprenant, dans un milieu physiologiquement acceptable,

a) au moins un substrat de la Cytochrome C oxydase et/ou un agent augmentant l'expression dudit substrat, et
b) au moins un composé émettant et/ou filtrant, notamment sous exposition lumineuse, au moins un rayonnement lumineux présentant au moins une longueur d'onde prédominante activant la Cytochrome C oxydase.

Ledit composé émettant et/ou filtrant, notamment sous exposition lumineuse, un rayonnement présentant au moins une longueur d'onde prédominante activant la cytochrome C oxydase est tel que défini précédemment.
En particulier, il peut être choisi parmi les métabolites et actifs émettant et/ou filtrant, notamment sous exposition lumineuse, un rayonnement de longueur d'onde prédominante allant de 550 à 800nm, de préférence de 620 à 700nm, et plus préférentiellement de 640 à 680nm.
A titre d'exemples, on peut citer notamment les anthocyanes de couleur rouge, tels que la carthamine, la cyanidine, la delphinidine, la pelargonine, la bétanine ; les polyphénols de couleur rouge ; le lycopène ; la braziléine, l'extrait de Rocou, la Garance, les pigments rouges, les complexes de fer III avec l'acide salicylique, l'octopirox ou le thiocyanate, les substances phosphorescentes rouges, les substances fluorescentes rouges, et leurs mélanges.
De préférence, on utilisera les anthocyanes de couleur rouge, le lycopène et leurs mélanges.
En particulier, le composé émettant et/ou filtrant, notamment sous exposition lumineuse, un rayonnement lumineux présentant au moins une longueur d'onde prédominante activant la cytochrome C oxydase est présent dans la composition en une teneur allant de 0,01 à 20% en poids par rapport au poids total de ladite composition.
Selon un mode préféré, le substrat de la cytochrome C oxydase est choisi parmi la cytochrome C, l'un de ses analogues cytochromes C1, C2 ou C3, et leurs mélanges.
En particulier, le substrat de la Cytochrome C oxydase et/ou un agent augmentant l'expression dudit substrat est présent dans la composition en une teneur allant de 0,001 à 30% en poids par rapport au poids total de ladite composition.
La composition peut être une composition cosmétique ou pharmaceutique.
De préférence, il s'agira d'une composition cosmétique.
Selon un premier mode de réalisation, la composition est destinée à une application topique sur la peau et/ou le cheveu.
Selon un autre mode de réalisation, la composition est destinée à une administration par voie orale.
La composition pourra encore comprendre un agent favorisant la pénétration desdits composés, tels que des solvants , des agents desquamants, et leurs mélanges. L'homme du métier veillera à ce que le choix de ces ingrédients additionnels soit compatible avec l'application d'un dispositif émettant un rayonnement lumineux activant la cytochrome C oxydase et n'altère pas les propriétés recherchées.
L'invention a encore pour objet un kit comprenant :

a) une composition comprenant au moins un substrat de la Cytochrome C oxydase et/ou un agent augmentant l'expression dudit substrat, et au moins un composé capable d'émettre et/ou de filtrer un rayonnement lumineux présentant au moins une longueur d'onde prédominante activant la cytochrome C oxydase ;
b) un premier dispositif permettant audit composé présent dans la première composition d'émettre et/ou de filtrer ledit rayonnement lumineux présentant au moins une longueur d'onde prédominante activant la cytochrome C oxydase.

Selon une alternative, l'invention concerne un kit comprenant :

a) une première composition comprenant au moins un substrat de la Cytochrome C oxydase et/ou un agent augmentant l'expression dudit substrat ;
b) une seconde composition comprenant au moins un composé capable d'émettre et/ou de filtrer un rayonnement lumineux présentant au moins une longueur d'onde prédominante activant la cytochrome C oxydase;
c) éventuellement, un premier dispositif permettant audit composé présent dans la seconde composition d'émettre et/ou de filtrer ledit rayonnement lumineux présentant au moins une longueur d'onde prédominante activant la cytochrome C oxydase.

Dans le cas d'un composé capable d'émettre le rayonnement lumineux activant la cytochrome C oxydase, le premier dispositif excite ledit composé de manière à lui faire émettre ledit rayonnement lumineux activant la cytochrome C oxydase.
Ledit premier dispositif peut être notamment :

- un dispositif émettant un rayonnement lumineux, présentant des longueurs d'onde de la lumière visible, UV et/ou

proche IR,

- une composition distincte destinée à être appliquée sur la peau et/ou le cheveu, comprenant un composé additionnel nécessaire à la réaction de chimiluminescence, de bioluminescence ou de thermoluminescence avec ledit composé.

En particulier, le premier dispositif peut être un dispositif émettant un rayonnement lumineux présentant des longueurs d'onde de la lumière visible et/ou de l'UV, en fonction de la nature du composé présent dans la seconde composition. Le dispositif pourra émettre un rayonnement lumineux choisi parmi la lumière blanche, une lumière colorée, et une lumière UV. De préférence il s'agira de la lumière visible et en particluier de la lumière rouge.
Dans le cas d'un composé capable de filtrer le rayonnement lumineux activant la cytochrome C oxydase, le premier dispositif est généralement un rayonnement lumineux qui est filtré par ledit composé en un rayonnement lumineux activant la cytochrome C oxydase.
Selon un mode particulier, les kits décrits précédemment peuvent comprendre en outre un second dispositif émettant un rayonnement lumineux présentant au moins une longueur d'onde prédominante activant la cytochrome C oxydase.
L'invention porte encore sur un kit comprenant :

a) une composition comprenant au moins un substrat de la Cytochrome C oxydase et/ou un agent augmentant l'expression dudit substrat ;
b) un dispositif émettant un rayonnement lumineux présentant au moins une longueur d'onde prédominante activant la cytochrome C oxydase.

Le dispositif émettant un rayonnement lumineux présentant au moins une longueur d'onde prédominante activant la cytochrome C oxydase est notamment choisi parmi un dispositif émettant de la lumière blanche associée à un filtre spécifique laissant passer ledit rayonnement lumineux activant la cytochrome C oxydase (en particulier un rayonnement lumineux présentant au moins une longueur d'onde prédominante correspondant au spectre d'émission de la lumière rouge et/ou infra-rouge), les lasers, l'IPL, les LED, et leurs combinaisons.
Le substrat de la Cytochrome C oxydase et/ou un agent augmentant l'expression dudit substrat sont tels que décrits précédemment.
Des exemples de composés émettant et/ou filtrant, notamment sous exposition lumineuse, un rayonnement présentant au moins une longueur d'onde prédominante activant la cytochrome C oxydase sont décrits précédemment.
Le kit selon l'invention peut être un kit cosmétique ou un kit pharmaceutique.
De préférence, il s'agira d'un kit cosmétique.
Les compositions constitutives de ces kits peuvent être des compositions destinées à une administration par voie topique ou orale.
De préférence, il s'agira de compositions destinées à une administration par voie topique.

**GALENIQUE**

Les compositions mises en oeuvre dans le procédé, la composition ou le kit selon l'invention, tels que décrits précédemment, sont destinées à une application topique et/ou orale.
La composition comprend généralement un milieu physiologiquement acceptable, c'est-à-dire compatible avec la peau et/ou ses phanères. Il s'agit de préférence d'un milieu cosmétiquement acceptable, c'est-à-dire qui présente une odeur, une couleur et un toucher agréables et qui ne génère pas d'inconforts (picotements, rougeurs, tiraillements) inacceptables, susceptibles de détourner la consommatrice d'utiliser cette composition. Pour une administration par voie orale, en particulier en 'cosmétique orale', elle peut se présenter notamment sous forme de capsules, de gélules, dragées, de granulés, de pâte à mâcher, de gels, de sirops buvables,de comprimés ou de toute autre forme connue de l'homme du métier.
De préférence, il s'agit d'une composition topique .
Par composition topique, on entend une composition destinée à une application locale sur toute surface du corps, incluant, la peau, les cheveux.
Pour une application topique sur la peau, la composition peut avoir la forme d'une solution aqueuse, hydroalcoolique ou huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, d'un produit anhydre liquide, pâteux ou solide ou encore de microémulsions, de microcapsules, de microparticules ou d'une dispersion vésiculaire de type ionique (liposomes ou oléosomes) et/ou non ionique (niosomes) et/ou d'une dispersion de minuscules sphères.
On peut également envisager une composition sous forme de mousse ou encore sous forme de spray ou d'aérosol

comprenant alors un agent propulseur sous pression ; ou encore sous forme de patch ou tampon imbibé.

La composition peut ainsi se présenter sous forme d'une lotion, sérum, lait, crème H/E ou E/H, gel, onguent, pommade, poudre, baume, patch, tampon imbibé, savon, pain, mousse.

Selon la zone du corps visée et l'intensité d'application désirée, l'homme de métier pourra choisir parmi différentes formes de composition :

- une composition destinée à restée appliquée sur la peau même après l'exposition au rayonnement lumineux présentant au moins une longueur d'onde prédominante activant la cytochrome C oxydase, cette composition peut être une dispersion du type lotion ou gel, une émulsion de consistance liquide ou semi-liquide du type lait, obtenue par dispersion d'une phase grasse dans une phase aqueuse (H/E) ou inversement (E/H), ou une suspension ou émulsion de consistance molle, semi-solide ou solide du type crème ou gel, ou encore des émulsions multiples (E/H/E ou H/E/H), une microémulsion, une dispersion vésiculaire de type ionique et/ou non ionique, ou une dispersion cire/phase aqueuse ;
- une composition qui ne reste en contact avec la peau que pendant la durée de l'exposition audit rayonnement lumineux activant la cytochrome C oxydase, telle qu'un masque, sous forme de crème que l'utilisateur peut appliquer spécifiquement sur les zones à traiter puis retirer ensuite ; ou un patch imbibé d'un substrat ; sous cette forme d'administration, les masques ou patchs doivent être suffisamment transparents pour laisser passer la lumière activant la cytochrome C oxydase.

L'invention va maintenant être illustrée par les exemples non limitatifs suivants.

**Figure 1 :** montage du test *in vitro* de l'activation de la cytochrome C oxydase par la lumière rouge.
**Figure 2 :** spectre d'émission de la lumière rouge utilisé dans les exemples

**Exemple 1- Activation de la Cytochrome C oxydase par un rayonnement lumineux spécifique (spectre d'émission de la lumière rouge)**

L'exemple suivant mesure l'effet stimulateur d'un rayonnement lumineux présentant une longueur d'onde prédominante allant de 550 à 800nm, en particulier de 620 à 700nm sur l'activité de la cytochrome C oxydase. L'exposition de cette enzyme audit rayonnement lumineux stimule la production de molécules d'ATP et d'eau selon la réaction chimique suivante :

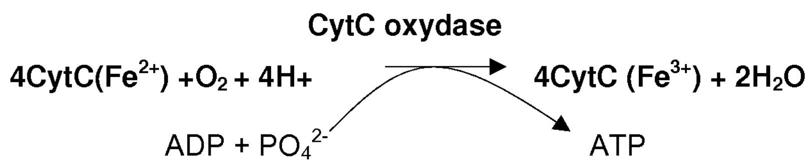


Description du protocole

Principe général : Le principe de ces travaux est de comparer l'activité de la cytochrome C oxydase avec ou sans exposition préalable à un rayonnement lumineux présentant une longueur d'onde allant de 620 à 700nm.

Matériels et réactifs

- source de lumière, avec fibre optique (diamètre interne 3mm) et Photomètre IL-1700 + sonde SED 033 (#6600) : produit un rayonnement lumineux dont le spectre d'émission est présenté à la **figure 2** ;
- Cytochrome c (0.22 mM) et Cytochrome c Oxidase (0.32 U/ml) : kit CYTOCOX1 de SIGMA

La réduction de la protéine se fait par ajout de la solution de 0.1 M DTT (dithiothreitol) soit une concentration finale de 0.5 mM.
On mesure l'absorbance à 550nm et à 565 nm: le ratio A550/A565 doit être compris entre 10 et 20 (contrôle la conformité des solutions initiales)

Mode exposition des enzymes

L'enzyme (forme poudre déshydratée ou solution d'enzyme) est disposée dans un tube Ependorf conique. La quantité est ajustée de manière à ce que l'ensemble des enzymes soit soumis aux rayonnements lumineux (longueur d'ondes comprises entre 620 et 700 nm) par une fibre optique de diamètre 3 mm. L'exposition lumineuse est continue et l'énergie fournie par la lumière est 0.45mW/$cm^2$ (voir **figure 1 :** schéma de montage et **figure 2 :** spectre d'émission de la lumière rouge utilisée dans les tests).
Les tubes Ependorf sont mis dans un bac à glace pendant toute la durée de l'exposition afin d'éviter un effet de la température. L'enzyme témoin (non exposée audit rayonnement lumineux) est préparée au même moment, de la même manière et protégée dudit rayonnement lumineux par un support empêchant l'apport de lumière extérieure (papier aluminium).
La cytochrome C oxydase est exposée au rayonnement lumineux (durée de 30 à 90min), soit une dose équivalente comprise entre 0.8 Joule/$cm^2$ et 2.4 Joule/$cm^2$, en solution à la concentration 0.15 U/ml dans un tampon TRIS-HCL pH 7 contenant 0.5 mM de sucrose. Après exposition, la cytochrome C oxydase est incubée avec son substrat, le ferrocytochrome C Fe2+ dans un mélange contenant 10 mM de TRIS HCL PH 7 et 120 mM de KCL.
L'activité de l'enzyme est évaluée dès le début de la réaction.

Mesure d'activité de l'enzyme

L'absorption du cytochrome C à 550 nm varie avec son état d'oxydation.
Cette propriété est la base de notre test de mesure.
Le Cytochrome C est réduit par le Dithiothreitol et réoxydé par la cytochrome C oxydase.
On dispose dans les eppendorfs 125 μl pour le témoin et 125 μl pour l'échantillon à tester.
L'échantillon à tester est alors placé sous rayonnement lumineux pendant une durée de 90 minutes.
On mesure alors la variation de l'absorbance A550/min.
L'activité enzymatique est alors calculée selon la formule suivante.

$$U/ml = (dA/min \times dil \times 1.1) / (vol\ d'enzyme) \times 21.84).$$

Le rayonnement lumineux émettant une longueur d'onde prédominante dans le rouge stimule significativement l'activité de la cytochrome C oxydase (+33%) pour une durée d'irradiation de 90 min, soit une dose équivalente à 2.4 Joule/$cm^2$.

**Exemple 2 - Exemples de formulations**

**Compositions associées à un dispositif**

Les formules décrites ci dessous seront appliquées sur le visage ou sur le corps juste avant une exposition prolongée (30 mn à 1h30) à un rayonnement lumineux présentant au moinsune longueur d'onde prédominante allant de 550 à 1000 nm, de préférence de 620 à 700nm. On utilisera par exemple un dispositif de type Omnilux™ system (633 nm) de la société Photo Therapeutics Ltd ou un dispositif de type Lightwave™ system (630nm et 880nm) de la société OPUSMED Inc

| ***Crème de soin de la peau:<br>émulsion huile dans eau*** | |
|---|---|
| Ammonium Polyacryldimethyltauramide (Hostacerin AMPS de Clariant) | 1.00% |
| Cyclohexasiloxane | 5.0% |
| Glycerine | 1.70% |
| Alcool stéarylique | 0.30% |
| Glyceryl stearate / PEG-100 stearate | 0.70% |
| Dimyristyl tartrate / cetearyl alcohol / C12-15 pareth-7 / PPG-25 laureth-25 | 0.50% |
| Gomme de Xanthan | 0,20 % |

(suite)

| | |
|---|---|
| ***Crème de soin de la peau: émulsion huile dans eau*** | |
| **Cytochrome C** | **0.5%** |
| Conservateurs | 0.50% |
| Eau | qsp 100 |
| | |
| ***Crème de soin de la peau: émulsion huile dans eau*** | |
| Ammonium Polyacryldimethyltauramide (Hostacerin AMPS de Clariant) | 1.00% |
| Cyclohexasiloxane | 5.0% |
| Glycerine | 1.70% |
| Alcool stéarylique | 0.30% |
| Glyceryl stearate / PEG-100 stearate | 0.70% |
| Dimyristyl tartrate / cetearyl alcohol / C12-15 pareth-7 / PPG-25 laureth-25 | 0.50% |
| Gomme de Xanthan | 0,20 % |
| **Cytochrome C (en matière active)** | **0,0175%** |
| Conservateurs | 0.50% |
| Eau | qsp 100 |

**Patch iontophorétique**

Selon un autre mode de réalisation, un patch de référence commerciale IontopatchTM (Travanti Pharma, Mendota Heights, MN, USA) est appliqué sur une zone préalablement traitée avec l'une des crèmes ci-dessus.
Il est ensuite relié à un courant électrique pour délivrer un courant galvanique généré par une différence de potentiel de 1V et comportant deux électrodes, une anode en Zn et une cathode en AgCl et est exposé à un rayonnement lumineux présentant au moins une longueur d'onde prédominante allant de 550 à 1000nm.
Ce traitement est réalisé à raison d'une fois par jour pendant 30 à 45 minutes.

**Masque rouge (1)**

| Phase huileuse : | |
|---|---|
| Octyl dodecanol | 6% |
| Huile de noyau d'abricot | 6% |
| Triglycerides | 5% |
| Kaolin | 3% |
| Alcool cétylique | 2% |
| Acetate de vitamine E | 0.5% |
| Huile de palm hydrogénée | 6% |
| Fraction liquide de beurre de Karité | 5% |
| Phase aqueuse : | |
| Gomme de Xanthane | 0.4% |
| Cocoate de sucrose/stearate de de sorbitan (mélange vendu par la société ICL sous la dénomination Arlaton 21121 | 5.5% |
| Glycerine | 3% |
| Dipyridamole | 0.30% |
| Ethanol | 5% |
| **Cytochrome C** | **0,1%** |

(suite)

| Phase aqueuse : | |
|---|---|
| **Delphinidine** | **0,4%** |
| Parfum | 0.3% |
| Conservateur | qs |
| Eau | qsp100 |

**Masque rouge (2)**

| Phase huileuse : | |
|---|---|
| Octyl dodecanol | 6% |
| Huile de noyau d'abricot | 6% |
| Triglycerides | 5% |
| Kaolin | 3% |
| Alcool cétylique | 2% |
| Acetate de vitamine E | 0.5% |
| Huile de palm hydrogénée | 6% |
| Fraction liquide de beurre de Karité | 5% |
| Phase aqueuse : | |
| Gomme de Xanthane Cocoate de sucrose/stearate de de sorbitan (mélange vendu par la société ICL | 0.4% |
| sous la dénomination Arlaton 21121 | 5.5% |
| Glycerine | 3% |
| Dipyridamole | 0.30% |
| Ethanol | 5% |
| **Cytochrome C (en matière active)** | **0,00002%** |
| **Delphinidine** | **0,4%** |
| Parfum | 0.3% |
| Conservateur | qs |
| Eau | qsp100 |

Le masque est appliqué sur les zones de la peau à traiter, puis le sujet est placé sous une source de lumière blanche (naturelle ou électrique) pendant 45 minutes à 1 heure.

## Revendications

**1.** Procédé cosmétique destiné notamment à améliorer l'apparence de la peau et/ou du cheveu comprenant l'administration simultanée ou séquentielle :

a) d'au moins un substrat de la cytochrome C oxydase qui est choisi parmi la cytochrome C, l'un de ses analogues cytochromes C1, C2 ou C3, et leurs mélanges, formulé(s) dans une composition pour administration par voie topique appliquée sur la peau et/ou sur le cheveu et présent en une teneur allant de 0,01 à 1% en poids par rapport au poids total de ladite composition; et
b) d'au moins un rayonnement lumineux présentant au moins une longueur d'onde prédominante allant de 620 à 700 nm, qui est appliqué sur la peau et/ou le cheveu au niveau des zones de peau et/ou de cheveu traitées par ladite composition définie en a).

**2.** Procédé selon la revendication 1, dans lequel le rayonnement lumineux présente au moins une longueur d'onde prédominante allant de 640 à 680 nm.

**3.** Procédé selon l'une des revendications 1 ou 2, dans lequel le rayonnement lumineux présentant au moins une longueur d'onde prédominante allant de 620 à 700 nm est appliqué sur la peau et/ou le cheveu, en particulier au niveau des zones de peau et/ou de cheveu traitées par la composition comprenant au moins un substrat de la

cytochrome C oxydase, à une dose allant de 0,01 à 200 J/cm2, de préférence de 0,1 à 30 J/$cm^2$.

4. Procédé selon l'une quelconque des revendications précédentes, dans lequel le rayonnement lumineux présentant au moins une longueur d'onde prédominante allant de 620 à 700 nm est émis par un dispositif choisi notamment parmi un dispositif émettant de la lumière blanche associée à un filtre spécifique laissant passer ledit rayonnement lumineux présentant au moins une longueur d'onde prédominante allant de 620 à 700 nm; les lasers ; l'IPL ; les LED ; et leurs combinaisons.

5. Procédé selon l'une quelconque des revendications 1 à 3, dans lequel le rayonnement lumineux présentant au moins une longueur d'onde prédominante allant de 620 à 700 nm est émis par un composé choisi parmi les métabolites ou actifs émettant, notamment sous exposition lumineuse, une telle longueur d'onde prédominante allant de 620 à 720 nm, un composé filtrant la lumière pour laisser spécifiquement passer la ladite longueur d'onde prédominante allant de 620 à 720 nm, et leurs mélanges.

6. Procédé selon la revendication 5, dans lequel le composé émet et/ou filtre un rayonnement lumineux présentant au moins une longueur d'onde prédominante allant 640 à 680 nm.

7. Procédé selon la revendication 6, dans lequel ledit composé émettant et/ou filtrant ledit rayonnement lumineux présentant au moins une longueur d'onde prédominante allant de 620 à 700 nm est choisi parmi les anthocyanes de couleur rouge, tels que la carthamine, la cyanidine, la delphinidine, la pelargonine, la bétanine ; les polyphénols de couleur rouge ; le lycopène ; la braziléine, l'extrait de Rocou, la Garance, les pigments rouges, les complexes de fer III avec l'acide salicylique, l'octopirox ou le thiocyanate, les substances phosphorescentes rouges, les substances fluorescentes rouges, et leurs mélanges.

8. Procédé selon l'une quelconque des revendications 5 à 7, dans lequel ledit composé émettant et/ou filtrant, notamment sous exposition lumineuse, un rayonnement lumineux présentant au moins une longueur d'onde prédominante allant de 620 à 700 nm, est . soit présent dans la composition contenant le substrat de la cytochrome C oxydase, . soit présent dans une composition distincte.

9. Procédé selon l'une quelconque des revendications précédentes, visant à améliorer l'hydratation de la peau et/ou du cheveu.

10. Procédé selon l'une quelconque des revendications 1 à 8, visant à améliorer la fonction barrière cutanée.

11. Procédé selon l'une quelconque des revendications 1 à 8, visant à prévenir et/ou lutter contre les signes du vieillissement de la peau et/ou du cheveu.

12. Procédé selon l'une quelconque des revendications 1 à 8, visant à atténuer les irrégularités visibles ou tactiles de la surface de la peau, en particulier atténuer les rides et ridules, atténuer les tâches cutanées, diminuer les altérations du microrelief et/ou lisser la peau, favoriser la régénération du tissu cutané, éclaircir le teint et/ou améliorer l'aspect terne du teint, maintenir et/ou améliorer les propriétés biomécaniques de la peau (élasticité, fermeté, tonicité), maintenir et/ou améliorer l'hydratation de la peau, et/ou maintenir et/ou améliorer le grain de la peau.

13. Procédé selon l'une quelconque des revendications 1 à 8, visant à traiter le ralentissement de la pousse des cheveux, leur grisonnement, la diminution de leur diamètre, et de leur vigueur.

14. Composition topique comprenant, dans un milieu physiologiquement acceptable,

   a) au moins un substrat de la Cytochrome C oxydase qui est choisi parmi la cytochrome C, l'un de ses analogues cytochromes C1, C2 ou C3, et leurs mélanges , et
   b) au moins un composé émettant et/ou filtrant, notamment sous exposition lumineuse, un rayonnement lumineux présentant au moins une longueur d'onde prédominante allant de 620 à 700 nm.

15. Composition selon la revendication précédente, dans laquelle ledit composé émettant et/ou filtrant, notamment sous exposition lumineuse, un rayonnement lumineux présentant au moins une longueur d'onde prédominante allant de 620 à 700 nm est tel que défini dans l'une quelconque des revendications 5 à 7.

16. Kit comprenant :

a. une première composition comprenant au moins un substrat de la Cytochrome C oxydase qui est choisi parmi la cytochrome C, l'un de ses analogues cytochromes C1, C2 ou C3, et leurs mélanges,

- au moins un composé capable d'émettre et/ou de filtrer un rayonnement lumineux présentant au moins une longueur d'onde prédominante allant de 620 à 700 nm qui est soit présent dans ladite première composition, soit présent dans une seconde composition;

b. un dispositif permettant audit composé présent dans la première ou la seconde composition d'émettre et/ou de filtrer ledit rayonnement lumineux présentant au moins une longueur d'onde prédominante allant de 620 à 700 nm.

## Patentansprüche

1. Kosmetisches Verfahren, das insbesondere dazu bestimmt ist, das Aussehen der Haut und/oder der Haare zu verbessern, das das gleichzeitige oder aufeinander folgende Verabreichen von:

   a) mindestens einem Cytochrom-C-oxydase-Substrat, das aus Cytochrom-C, einem seiner Cytochrom-C1-, -C2- oder -C3-Analoga und deren Gemischen ausgewählt ist, das in einer Zusammensetzung zur topischen Verabreichung formuliert ist, die auf die Haut und/oder die Haare angewendet wird, und in einem Anteil von 0,01 bis 1 Gew.-%, bezogen auf das Gesamtgewicht der Zusammensetzung, vorhanden ist; und
   b) mindestens einer Lichtstrahlung, die mindestens eine vorwiegende Wellenlänge von 620 bis 700 nm aufweist, die auf die Haut und/oder die Haare auf den Bereichen der Haut und/oder der Haare angewendet wird, die mit der in a) definierten Zusammensetzung behandelt wurden,

   umfasst.

2. Verfahren nach Anspruch 1, wobei die Lichtstrahlung mindestens eine vorwiegende Wellenlänge von 640 bis 680 nm aufweist.

3. Verfahren nach einem der Ansprüche 1 oder 2, wobei die Lichtstrahlung, die mindestens eine vorwiegende Wellenlänge von 620 bis 700 nm aufweist, auf die Haut und/oder die Haare, insbesondere auf die Bereiche der Haut und/oder der Haare, die mit der Zusammensetzung behandelt wurden, die mindestens ein Cytochrom-C-oxydase-Substrat umfasst, in einer Dosis von 0,01 bis 200 J/cm$^2$, bevorzugt von 0,1 bis 30 J/cm$^2$ angewendet wird.

4. Verfahren nach einem der vorhergehenden Ansprüche, wobei die Lichtstrahlung, die mindestens eine vorwiegende Wellenlänge von 620 bis 700 nm aufweist, von einer Vorrichtung emittiert wird, die insbesondere aus einer Vorrichtung, die weißes Licht emittiert, in Kombination mit einem spezifischen Filter, der die Lichtstrahlung durchlässt, die mindestens eine vorwiegende Wellenlänge von 620 bis 700 nm aufweist; Lasern; IPL; LEDs; und deren Kombinationen ausgewählt ist.

5. Verfahren nach einem der Ansprüche 1 bis 3, wobei die Lichtstrahlung, die mindestens eine vorwiegende Wellenlänge von 620 bis 700 nm aufweist, von einer Verbindung emittiert wird, die aus Metaboliten oder Wirkstoffen ausgewählt ist, die, insbesondere bei Lichtexposition, eine solche vorwiegende Wellenlänge von 620 bis 700 nm emittieren, einer Verbindung, die das Licht filtert, um spezifisch die vorwiegende Wellenlänge von 620 bis 700 nm durchzulassen, und deren Gemischen.

6. Verfahren nach Anspruch 5, wobei die Verbindung eine Lichtstrahlung, die mindestens eine vorwiegende Wellenlänge von 640 bis 680 nm aufweist, emittiert und/oder filtert.

7. Verfahren nach Anspruch 6, wobei die Verbindung, die die Lichtstrahlung emittiert und/oder filtert, die mindestens eine vorwiegende Wellenlänge von 620 bis 700 nm aufweist, aus roten Anthocyanen, wie beispielsweise Carthamin, Cyanidin, Delphinidin, Pelargonin, Betanin; roten Polyphenolen; Lycopen; Brazilein, Annato-Extrakt, Krapp, roten Pigmenten, Eisen-III/Salicylsäure-Komplexen, Octopirox oder Thiocyanat, rot phosphoreszierenden Substanzen, rot fluoreszierenden Substanzen und deren Gemischen ausgewählt ist.

8. Verfahren nach einem der Ansprüche 5 bis 7, wobei die Verbindung, die, insbesondere bei Lichtexposition, eine Lichtstrahlung emittiert und/oder filtert, die mindestens eine vorwiegende Wellenlänge von 620 bis 700 nm aufweist,

. entweder in der Zusammensetzung vorhanden ist, die das Cytochrom-C-oxydase-Substrat enthält,
. oder in einer anderen Zusammensetzung vorhanden ist.

**9.** Verfahren nach einem der vorhergehenden Ansprüche, das darauf gerichtet ist, den Feuchtigkeitshaushalt der Haut und/oder der Haare zu verbessern.

**10.** Verfahren nach einem der Ansprüche 1 bis 8, das darauf gerichtet ist, die Barrierefunktion der Haut zu verbessern.

**11.** Verfahren nach einem der Ansprüche 1 bis 8, das darauf gerichtet ist, die Anzeichen der Alterung der Haut und/oder der Haare zu verhindern und/oder zu bekämpfen.

**12.** Verfahren nach einem der Ansprüche 1 bis 8, das darauf gerichtet ist, die sichtbaren oder fühlbaren Unregelmäßigkeiten der Hautoberfläche abzumildern, insbesondere Falten und Fältchen abzumildern, Pigmentflecken abzumildern, die Veränderungen des Mikroreliefs zu verringern und/oder die Haut zu glätten, die Regeneration des Hautgewebes zu fördern, den Teint aufzuhellen und/oder das blasse Aussehen des Teints zu verbessern, die biomechanischen Eigenschaften der Haut (Elastizität, Festigkeit, Tonus) zu erhalten und/oder zu verbessern, den Feuchtigkeitshaushalt der Haut zu erhalten und/oder zu verbessern und/oder die Hautstruktur zu erhalten und/oder zu verbessern.

**13.** Verfahren nach einem der Ansprüche 1 bis 8, das darauf gerichtet ist, die Verlangsamung des Wachstums der Haare, ihr Ergrauen, die Verringerung ihres Durchmessers und ihrer Spannkraft zu behandeln.

**14.** Topische Zusammensetzung, die in einem physiologisch verträglichen Medium Folgendes umfasst:

a) mindestens ein Cytochrom-C-oxydase-Substrat, das aus Cytochrom-C, einem seiner Cytochrom-C1-, -C2- oder -C3-Analoga und deren Gemischen ausgewählt ist, und
b) mindestens eine Verbindung, die, insbesondere bei Lichtexposition, eine Lichtstrahlung emittiert und/oder filtert, die mindestens eine vorwiegende Wellenlänge von 620 bis 700 nm aufweist.

**15.** Zusammensetzung nach dem vorhergehenden Anspruch, wobei die Verbindung, die, insbesondere bei Lichtexposition, eine Lichtstrahlung emittiert und/oder filtert, die mindestens eine vorwiegende Wellenlänge von 620 bis 700 nm aufweist, so ist, wie in einem der Ansprüche 5 bis 7 definiert.

**16.** Kit, umfassend:

a. eine erste Zusammensetzung, die mindestens ein Cytochrom-C-oxydase-Substrat umfasst, das aus Cytochrom-C, einem seiner Cytochrom-C1-, -C2- oder -C3-Analoga und deren Gemischen ausgewählt ist;

- mindestens eine Verbindung, die in der Lage ist, eine Lichtstrahlung zu emittieren und/oder zu filtern, die mindestens eine vorwiegende Wellenlänge von 620 bis 700 nm aufweist, die entweder in der ersten Zusammensetzung vorhanden ist oder in einer zweiten Zusammensetzung vorhanden ist;

b. eine Vorrichtung, die es der Verbindung, die in der ersten oder der zweiten Zusammensetzung vorhanden ist, ermöglicht, die Lichtstrahlung zu emittieren und/oder zu filtern, die mindestens eine vorwiegende Wellenlänge von 620 bis 700 nm aufweist.

## Claims

**1.** Cosmetic method intended in particular to improve the appearance of the skin and/or hair comprising the simultaneous or sequential administration:

a) of at least one cytochrome C oxidase substrate which is chosen from cytochrome C, one of its analogues cytochrome C1, C2 or C3, and their mixtures, which is (are) formulated in a composition for topical administration applied to the skin and/or to the hair and present in a content ranging from 0.01 to 1% by weight, with respect to the total weight of the said composition; and
b) of at least one light radiation exhibiting at least one predominant wavelength ranging from 620 to 700 nm, which is applied to the skin and/or hair, on the areas of skin and/or hair treated with the said composition defined

in a).

2. Method according to Claim 1, in which the light radiation exhibits at least one predominant wavelength ranging from 640 to 680 nm.

3. Method according to either of Claims 1 and 2, in which the light radiation exhibiting at least one predominant wavelength ranging from 620 to 700 nm is applied to the skin and/or hair, in particular to the areas of skin and/or hair treated with the composition comprising at least one cytochrome C oxidase substrate, at a dose ranging from 0.01 to 200 J/cm$^2$, preferably from 0.1 to 30 J/cm$^2$.

4. Method according to any one of the preceding claims, in which the light radiation exhibiting at least one predominant wavelength ranging from 620 to 700 nm is emitted by a device chosen in particular from a device emitting white light in combination with a specific filter which allows the said light radiation exhibiting at least one predominant wavelength ranging from 620 to 700 nm to pass; lasers; IPL; LEDs; and their combinations.

5. Method according to any one of Claims 1 to 3, in which the light radiation exhibiting at least one predominant wavelength ranging from 620 to 700 nm is emitted by a compound chosen from metabolites or active principles emitting, in particular under exposure to light, such a predominant wavelength ranging from 620 to 700 nm, a compound which filters the light in order to specifically allow the said predominant wavelength ranging from 620 to 700 nm to pass, and their mixtures.

6. Method according to Claim 5, in which the compound emits and/or filters a light radiation exhibiting at least one predominant wavelength ranging from 640 to 680 nm.

7. Method according to Claim 6, in which the said compound emitting and/or filtering the said light radiation exhibiting at least one predominant wavelength ranging from 620 to 700 nm is chosen from red-coloured anthocyanins, such as carthamin, cyanidin, delphinidin, pelargonin or betanin; red-coloured polyphenols; lycopene; brazilein, annatto extract, madder, red pigments, complexes of iron(III) with salicylic acid, octopirox or thiocyanate, red phosphorescent substances, red fluorescent substances and their mixtures.

8. Method according to one of Claims 5 to 7, in which the said compound emitting and/or filtering, in particular under exposure to light, a light radiation exhibiting at least one predominant wavelength ranging from 620 to 700 nm is: either present in the composition comprising the cytochrome C oxidase substrate, or present in a separate composition.

9. Method according to any one of the preceding claims, which is targeted at improving the hydration of the skin and/or hair.

10. Method according to any one of Claims 1 to 8, which is targeted at improving the skin barrier function.

11. Method according to any one of Claims 1 to 8, which is targeted at preventing and/or combating signs of aging of the skin and/or hair.

12. Method according to any one of Claims 1 to 8, which is targeted at alleviating visible or tactile irregularities in the surface of the skin, in particular alleviating wrinkles and fine lines, alleviating skin blemishes, reducing detrimental changes in the microrelief and/or smoothing the skin, promoting the regeneration of skin tissue, lightening the complexion and/or improving the lifeless appearance of the complexion, maintaining and/or improving the biomechanical properties of the skin (elasticity, firmness, tonicity), maintaining and/or improving the hydration of the skin and/or maintaining and/or improving the texture of the skin.

13. Method according to one of Claims 1 to 8, which is targeted at treating the slowing down in hair growth, the greying of hair, the reduction in the diameter of the hairs and the reduction in the vigour of the hairs.

14. Topical composition comprising, in a physiologically acceptable medium,

a) at least one cytochrome C oxidase substrate which is chosen from cytochrome C, one of its analogues cytochrome C1, C2 or C3, and their mixtures, and
b) at least one compound emitting and/or filtering, in particular under exposure to light, a light radiation exhibiting

at least one predominant wavelength ranging from 620 to 700 nm.

**15.** Composition according to the preceding claim, in which the said compound emitting and/or filtering, in particular under exposure to light, a light radiation exhibiting at least one predominant wavelength ranging from 620 to 700 nm is as defined in any one of Claims 5 to 7.

**16.** Kit comprising:

a) a first composition comprising at least one cytochrome C oxidase substrate which is chosen from cytochrome C, one of its analogues cytochrome C1, C2 or C3, and their mixtures,
at least one compound capable of emitting and/or filtering a light radiation exhibiting at least one predominant wavelength ranging from 620 to 700 nm which is either present in the said first composition or present in a second composition;
b) a device which makes it possible for the said compound present in the first or the second composition to emit and/or filter the said light radiation exhibiting at least one predominant wavelength ranging from 620 to 700 nm.

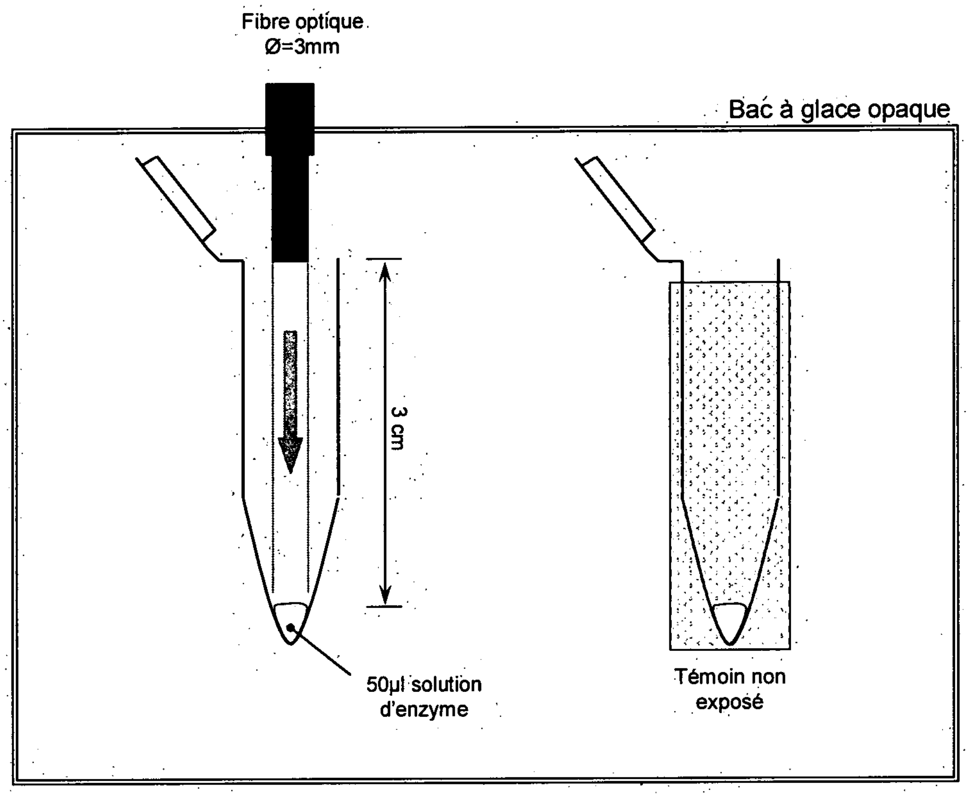
Fibre optique
Ø=3mm
Bac à glace opaque
3 cm
50µl solution
d'enzyme
Témoin non
exposé

FIGURE 1

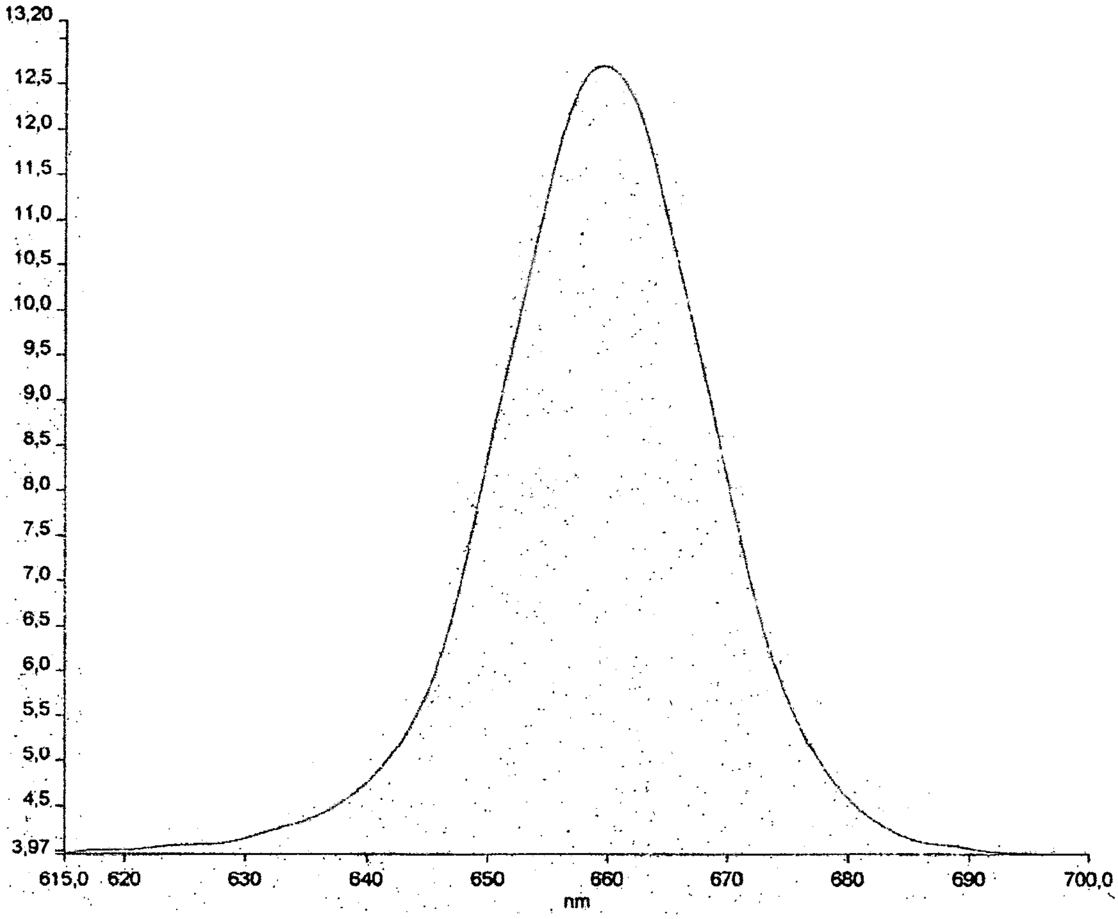
13,20
12,5
12,0
11,5
11,0
10,5
10,0
9,5
9,0
8,5
8,0
7,5
7,0
6,5
6,0
5,5
5,0
4,5
3,97
615,0
620
630
640
650
660
670
680
690
700,0
nm

FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004075985 A
- FR 0758017
- EP 1754968 A2
- EP 1369681 A1


### Littérature non-brevet citée dans la description


- **G. S. GERHARD et al.** *Mechanisms of Ageing and development,* 2002, vol. 123, 155-166
- **PETER BAMFIELD.** Chromic Phenomena, Technological Applications of Colour Chemistry. The Royal Society of Chemistry, 2001